# EUROPÄISCHE PATENTSCHRIFT

(11) **EP 0 735 883 B1**
(45) Veröffentlichungstag und Bekanntmachung des Hinweises auf die Patenterteilung: **02.11.2006**
(21) Anmeldenummer: 95905574.0
(22) Anmeldetag: 22.12.1994
(51) Int. Cl.: A61K 31/57, A61K 31/565, A61P 15/18

(54) **ZUSAMMENSETZUNG FÜR DIE EMPFÄNGNISVERHÜTUNG UMFASSEND EIN ESTROGEN UND EIN GESTAGEN**
COMPOSITION FOR CONTRACEPTION COMPRISING AN ESTROGEN AND A GESTAGEN
COMPOSITION CONTRACEPTIVE COMPRENANT UN ESTROGENE ET UN GESTAGENE

(30) Priorität: 22.12.1993 DE 4344462
(43) Veröffentlichungstag der Anmeldung: 09.10.1996
(60) Teilanmeldung: 06076732.4
(73) Patentinhaber: Schering Aktiengesellschaft, 13353 Berlin (DE)
(72) Erfinder: SPONA, Jürgen Vitalogic, 1020 Wien (AT); DÜSTERBERG, Bernd, 16727 Oberkrämer / OT Bärenklau (DE); LÜDICKE, Frank, 82024 Taufkirchen (DE); FEICHTINGER, Wilfried, 1130 Wien (AT); ELSTEIN, Max, Manchester M20 2QT (GB)
(74) Vertreter: Plougmann & Vingtoft A/S
(86) Internationale Anmeldenummer: PCT/EP1994/004274
(87) Internationale Veröffentlichungsnummer: WO 1995/017194

(56) Entgegenhaltungen:
- CONTRACEPTION, 43 (1). 1991. 23-33., MELIS G B ET AL 'A COMPARATIVE STUDY ON THE EFFECTS OF A MONOPHASIC PILL CONTAINING DESOGESTREL PLUS 20 MUG ETHYNYLESTRADIOL A TRIPHASIC COMBINATION CONTAINING LEVONORGESTREL AND A MONOPHASIC COMBINATION CONTAINING GESTODENE ON COAGULATORY FACTORS'
- GENAZZANI, A. R., F. PETRAGLIA AND A. VOLPE (ED.). PROGRESS IN GYNECOLOGY AND OBSTETRICS;SECOND EUROPEAN WINTER CONFERENCE IN GYNECOLOGY AND OBSTETRICS, MADONNA DI CAMPIGLIO, ITALY, MARCH 1989. XXVI+944P. PARTHENON PUBLISHING GROUP: CARNFORTH, ENGLAN, BILOTTA P ET AL 'MULTICENTER CLINICAL TRIAL ON THE NEW ORAL CONTRACEPTIVE CONTAINING 20 MUG ETHYNYLESTRADIOL'

## Beschreibung

Die vorliegende Erfindung betrifft die gemeinsame Verwendung von Estrogenen und Gestagenen zur Herstellung eines monophasischen Kombinationspräparates für die orale Kontrazeption und eine entsprechende, dieses monophasische Kombinationspräparat enthaltende Packung.

Kombinationspräparate zur oralen Kontrazeption sind bereits bekannt, beispielsweise Femovan^{(R)} [DE-PS 2 546 062] oder Marvelon^{(R)} [DE-OS 2 361 120]. Diese Präparate bestehen aus 21 wirkstoffhaltigen (Estrogen/Gestagen) Dosierungseinheiten und 7 wirkstofffreien Dragees (Blindpillen; Placebos). Die täglich zu verabreichende Dosis ist jeweils gleich hoch (sogenannte Einphasen-Präparate) und bewirkt in der gesamten Einnahmezeit und in der Einnahmepause oder während der Einnahme der Placebos den erwünschten kontrazeptiven Effekt. Eine - in den meisten Präparaten - 7tägige Unterbrechung der Einnahme wirkstoffhaltiger Dosierungseinheiten wurde bis vor kurzem für notwendig gehalten, um eine zuverlässige Entzugsblutung auszulösen und damit eine zufriedenstellende Zykluskontrolle zu erreichen.

Andere Präparate, die mehr als 21, einen estrogenen und gestagenen Wirkstoff enthaltende, Dosierungseinheiten aufweisen und in denen die Einnahmepause teilweise (Ijzerman, Pasquale) oder ganz (Kuhl) durch estrogenhaltige Dosierungseinheiten überbrückt wird. Dabei ist es möglich, daß das ansonsten in oralen Kontrazeptiva enthaltene synthetische Estrogen Ethinylestradiol teilweise oder vollständig durch ein konjugiertes Estrogen, vorzugsweise Estradiol, ersetzt ist.

Niedrig dosierte, triphasische Kombinationspräparate zur hormonalen Kontrazeption, die 21 bis 24 bzw. 24 tägliche Estrogen/Gestagen-Dosierungseinheiten enthalten, gehen bereits aus den EP-As 0491 438 und 0 491 415 hervor, Gemäß der Lehre der EP-A 0 491 438 werden zunächst beginnend mit Tag 1 des Menstruationzyklus die zur Vervollständigung des im allgemeinen 28 tägigen Zyklus vorgesehenen Placebos eingenommen oder es ist zunächst ein pillen freies Intervall ohne Einnahme jeglicher kontrazeptiver Steroide vorgesehen.

Ein Kombinationspräparat zur Substitutionstherapie und Kontrazeption für Frauen in der Praemenopause (ab etwa dem 40. Lebensjahr) ist aus der EP-A-0 253 607 bekannt. Dieses Kombinotionspidparat enthält ein Estrogen aus der Gruppe
17β-Estradiol,
Ethinyleströdiol und
Mestranol
sowie ein Gestagen aus der Gruppe
Levonorgestrel,
Gestoden,
Desogestrel,
3-Ketodesogestrel und
Norethisteron.

Eine so gewählte Zusammensetzung soll hormonelle Unregelmäßigkeiten in der Übergangsphase der Praemenopause ausgleichen und die durch die hormonelle Umstellung des weiblichen Organismus in dieser Phase bedingten Beschwerden lindern helfen. Gleichzeitig gewährleistet eine derartige Zusammensetzung einer praemenopausalen Frau den in diesem Lebensalter noch nötigen kontrazeptiven Schutz.

Die Entwicklung neuer oraler Kontrazeptiva für Frauen im fertilen Alter vor der Praemenopause war während der letzten zwanzig Jahre vor allem durch die Reduktion der Estrogen- und Geslogendosierungen gekennzeichnet.

Die Verringerung der täglichen Hormondosis wurde mit der Erwartung verbunden, die Häufigkeit von unerwünschten Nebenwirkungen zu minimieren. Inzwischen erhobene epidemiologische Daten bestätigen den erwünschten Trend zur besseren Verträglichkeit niedriger dosierter Präparate bezogen auf kardiovaskuläre Komplikationen [(1.) Thorogood M, Oral Contraceptives and Cardiovascular Disease. An Epidemiologic Overview; Pharmacoepidemiology and Drug Safety, Vol 2: 3-16 (1993); (2.) Gerstman B B, Piper J M, Tomita D K, Ferguson W J, Stadel B V, Lundin F E; Oral Contraceptive Estrogen Dose and the Risk of Deep Venous Thromboembolic Disease, Am J E, Vol. 133, No 1, 32-36 (1981); (3.) Lidegaard O, Oral contraception and rist of a cerebral thromboembolic attack: results of a case-Control study; BMJ Vol 306, 956-63 (1993); (4.) Vessey M, Mant D. Smith A, Yeates D., Oral contraceptives and venous thromboembolism: findings in a large prospective study; BMJ, Vol 292, (1988); (5.) Mishell D R, Oral Contraception: Past, Present, and Future Perspectives; Int J Fertil, 36 Suppl.,7 - 18 (1991)].

Es wird angenommen, daß vor allem zwischen der Höhe der Estrogendosis und der inzidenz kordiovoskulöter Erkrankungen ein Zusammenhang besteht. Einer extremen Verringerung der täglichen Estrogendosis steht jedoch der Erholt der kontrazeptiven Wirksamkeit entgegen. Obwohl die ovulationshemmende Wirkung der niedrig dosierten oralen Kontrazeptiva vorwiegend durch die gestagene Komponente hervorgerufen wird, leistet auch die estrogene Komponente einen erheblichen Beitrag zur zentralen Hemmwirkung und zur ovariellen Suppression (Ovulationshemmung). Darüber hinaus darf die tägliche Estrogendosis Grenzdosisbereiche nicht unterschreiten, damit eine zufriedenstellende Zykluskontrolle gewährleistet werden kann (Der Frauenarzt; 34, 7; 793(1993)).

Die zur Zeit auf dem Markt befindliche niedrigste in einem oralen Kontrazeptivum enthaltene Estrogendosis beträgt 20 µg Ethinylestradiol, kombiniert mit 150 µg Desogestrel (Mercilon^{®}). Obwohl die Zykluskontrolle dieses Präparates im Vergleich zu Präparaten mit höherer Estrogendosis erwartungsgemäß etwas schlechter ist, weist die hohe Akzeptanzrate von Mercilon auf eine geringe klinische Relevanz dieses Nachteils hin. Ein klinisch bedeutsames Problem stellt jedoch die in mehreren Studien übereinstimmend gemachte Beobachtung einer geringeren ovariellen Suppression des 20 µg Ethinylestradiol enthalienden Präparates dar. Es kommt offensichtlich unter dieser sehr niedrigen Estrogendosis bei vielen Frauen zur Hercnreifung von Follikeln, die mit Ultrascholluntersuchungen bzw. Hormonuntersuchungen nachgewiesen werden konnten [(6.) Lunell NO, Cartström K, Zador G, Ovulation inhibition with a combined oral contraceptive containing 20 µg ethinylestradiol and 250 µg levonorgestrel; Acta Obstet Gynecol Scand Suppl. 88: 17-21 (1979); (7.) Mall-Haefeli M, Werner-Zodrow 1, Huber P R, Klinische Erfahrungen mit Mercilon und Marvelon unter besonderer Berücksichtigung der Ovar-Funktion; Geburtsh. und Frauenheilk. 51, 35-38, Georg Thieme Verlag, Stuttgart-New York (1991); (8.) Strobel E, Behandlung mit oralen Kontrazeptiva; Fortschr. Med. 110 Jg. Nr. 20 (1992); (9.) Letter to Editor, Contraception 45: 519-521 (1992); (10.) Teichmann A T, Brill K, Can Dose Reduction of Ethinylestradiol in OCs jeopardize Ovarian Suppression and Cycle Control? Abstract Book, VIIIth World Congress on Human Reproduction, Bali, Indonesia (1993)].

Die durchgeführten Hormonbestimmungen zeigten, daß es sich um funktionelle Granulosazellen handelt, die 17β-Estradiol sezernieren. Jeder Einnahmefehler bei Frauen mit deutlicher ovarieller Aktivität, also mit Follikelanreifungen. kann zu einem schnellen Anstieg der Gonodotrapin-Produktion führen. Die Voraussetzungen für eine Ovulation wären somit vorhanden. Man schätzt, daß etwa ein Drittel der Frauen orale Kontrazeptiva innerhalb eines Anwendungsjahres unregelmäßig einnimmt (Gynpress, 1. Jahrgang, Nr. 3, 1990). Das Risiko einer Schwangerschaft ist deshalb insbesondere bei Einnahmefehlern unter den 20 µg Ethinylestradiol-Präparaten hoch.

Melis et al. (Contraception, January 1991, Vol. 43, No.1) beschreibt die Verwendung einer monophasischen Pille mit 20 µg ethinyl estradiol plus 150 µg desogestrel zur Verhütung bei Frauen im Alter von 18-45 Jahren.

Die Aufgabe der vorliegenden Erfindung ist ein verbessertes monophasisches Kombinationspräparat für eine fertile Frau, die sich noch nicht in der Praemenopause befindet, enthaltend in jeder einzelnen Dosierungseinheit ein Estrogen und Gestagen, mit möglichst niedrigem Estrogengehalt in jeder einzelnen Dosierungseinheit. aber auch mit einem niedrigen Gesamthormongehalt pro Verabreichungszyklus.
Es wurde nun gefunden, daß eine ausgeprägte ovarielle Suppression ohne häufige Follikelanreifungen bei niedriger täglicher Estrogendosierung, niedriger Gesamtestrogen- sowie niedriger Gesomthormonmenge pro Verabrelchungszyklus erreicht werden kann durch die Verwendung einer Zusammensetzung umfassend ein Estrogen ausgewählt aus
2,0 bis 6,0 mg 17β-Estradiol und
0,015 bis 0.020 mg Ethinylestradiol:
und ein Gestagen ausgewählt aus
0,05 bis 0,075 mg Gestoden,
0.075 bis 0,125 mg Levonorgestrel,
0,06 bis 0,15 mg Desogestrel.
0.06 bis 0.15 mg 3-Keiodesogestrel.
0,2 bis 0.3 mg Norgestimat,
>0,35 bis 0.75 mg Norethisteron,
0,1 mg Drospirenon bis zu einer zu 0,075 mg Gestoden equivalenten Dosis von Drospirenon, und
0,1 mg Cyproteronacetat bis zu einer zu 0,075 mg Gestoden equivalenten Dosis von Cyproteranacetat,
für die Empfängnisverhütung für eine Frau im fertilen Alter, die die Praemenopause noch nicht erreicht hat, durch Verabreichung der Dosierungsform während 23 oder 24 Tagen, beginnend am Tag eins des Menstruaüonszyklus (erster Tag der Menstruationsblutung), gefolgt von 5 oder 4 pillenfreien oder Blindpillentagen, während insgesamt 28 lagen im Verabreichungszyklus.

Die einzelnen Dosierungsformen sollen dabei über alle 23 oder 24 Tage eine konstante Estrogen/Gestogenmenge enthalten.

Die Begriffe "Praemenopause" und "Menopause" werden im Rahmen vorliegender Erfindung im Sinne der konventionellen Definition gebraucht, siehe etwa "The Controversial Climacteric"; P.A. von Keep et al., Ed., MTP Press (1981). z.B. S. 9.

Die tägliche Hormondosis wird dabei auf sehr niedrigen Niveau gehalten, während die übliche 21 tägige Einnahme um zwei oder drei Tage verlängert ist. Die verbleibenden 5 oder 4 Tage eines Zyklus werden vorzugsweise durch Placebos überbrückt, um Einnahmefehler zu vermeiden, oder durch 5 oder 4 einnahmefreie Tage.

Gemäß einer bevorzugten Ausführungsform der vorliegenden Erfindung betrifft diese die Verwendung einer Zusammensetzung umfassend ein Estrogen ausgewählt aus
>2.0 bis 6,0 mg 17β-Estradiol und
0,020 mg Ethinylestradiol;
und ein Gestagen ausgewählt aus
>0,06 bis 0,075 mg Gestoden,
>0,100 bis 0.125 mg Levonorgestrel,
>0.10 bis 0,15 mg Desogestrel,
>0.10 bis 0.15 mg 3-Ketodesogestrel,
0,25 mg Drospirenon bis zu einer zu 0,075 mg Gestoden equivalenten Dosis von Drospirenon,
0,1 mg Cyproteronacetat bis zu einer zu 0,075 mg Gestoden equivalenten Dosis von Cyproteronacetat,
0,2 bis 0,3 mg Norgestimat und
0,50 bis 0,75 mg Norethisteron
für die Empfängnisverhütung wie vorstehend beschrieben.

Des weiteren betrifft die vorliegende Erfindung ein monophasisches Kombinationsprodukt für die orale Kontrazeption, weiches
(a) 23 oder 24 Dosierungseinheiten, jeweils enthaltend ein Estrogen ausgewählt aus
   >2,0 bis 6,0 mg 17β-Estradiol und
   0,020 mg Ethinylestradiol;
   und ein Gestagen ausgewählt aus
   0,25 mg Drospirenon bis zu einer zu 0,075 mg Gestoden equivalenten Dosis von Drospirenon,
   0,1 bis 0,2 mg Cyproteronacetat bis zu einer zu 0,075 mg Gestoden equivalenten Dosis von Cyproteronacetat,
   und
(b) 5 oder 4 Blindpillen oder andere indikationen, um anzuzeigen, daß die tägliche Verabreichung der 23 oder 24 Dosierungseinheiten von 5 oder 4 pillenfreien oder Blindpillentagen gefolgt sein sollen, umfaßt.

Weitere erfindungsgemäße Ausführungsformen ergeben sich aus den Merkmalen der Unteransprüche.

Ein gemäß der vorliegenden Erfindung besonders bevorzugtes Kombinationspräparat umfaßt 23 Dosierungseinheiten.

Die nachfolgend kurz beschriebene klinische Studie wurde mit Ethinylestradiol als Estrogen und Gestoden als Vertreter der Substanzklasse der erfindungsgemäß möglichen Gestagene durchgeführt.

Die 23tägige Gabe von 20 µg Ethinylestradiol in Kombination mit 75 µg Gestoden führt im Vergleich zur 21 tägigen Gabe zu einer stärkeren ovariellen Suppression. In einer doppelblinden, randomisierten Studie an gesunden Frauen mit normaler Ovarfunktion erhielten Gruppen von je 30 Probandinnen das Kombinationspräparat entweder einmal täglich über 21 oder 23 Tage sowie an 7 bzw. 5 Tagen Placebos (um den Doppelblind-Charakter der Studie zu gewährleisten).

Die Behandlung begann nach einem ovulatorischen, unbehandelten Vorzyklus am 1. Tag der Menstruationsblutung des darauffolgenden Zyklus und erstreckte sich insgesamt über drei Behandlungszyklen. Mit einem unbehandelten follow-up-Zyklus wurde die Studie abgeschlossen,

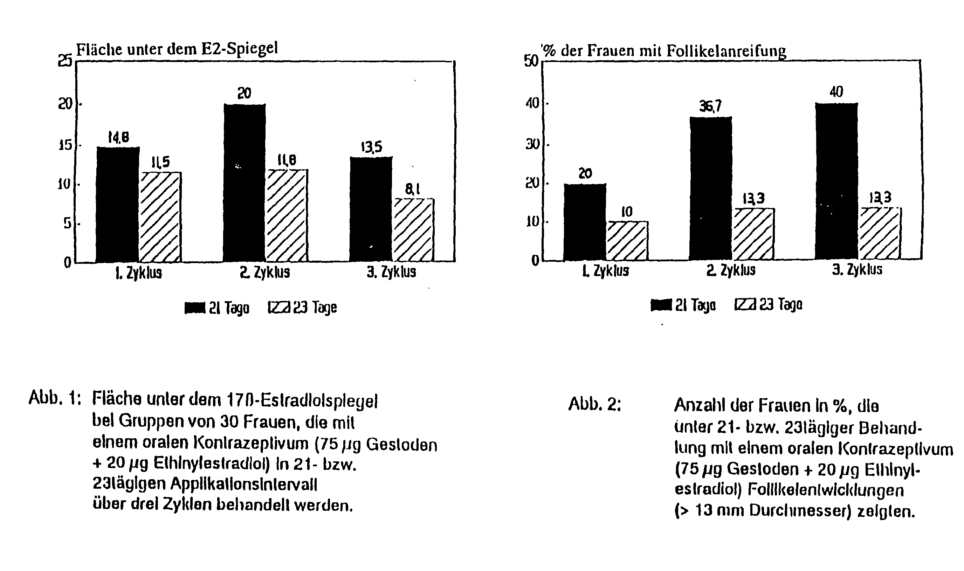

Die ovarielle Suppression wurde anhand der Höhe der endogenen 17β-Estradiolspiegel und der Größe follikularer Strukturen gemessen. Die Ergebnisse zeigen, daß die 17ß-Estradiolspiegel unter 23tägiger Einnahme des Prüfpräparates signifikant niedriger (p < 0.05) waren im Vergleich zur 21 tägigen Verabreichung (Abb. 1).

In Übereinstimmung mit diesem Befund war auch die Anzahl der Frauen mit Follikelanreifungen deutlich höher bei 21-maliger Gabe gegenüber der 23-maligen Applikation (Abb.2).

Das lediglich um zwei Tage verlängerte Einnahmeintervall, bewirkt überraschenderweise bei gleichbleibend niedriger Tagesdosis eine signifikant stärkere ovarielle Suppression. Das erfindungsgemäße Kombinationspräparat erreicht somit die bisher für Präparate mit einem täglichen Gehalt von 30 µg Ethinylestradiol bekannte Wirksamkeit, obwohl die tägliche Ethinylestradioldosis um 33 % niedriger und auch die Gesamtdosis pro Zyklus um 27 % geringer ist.

Die Vorteile eines über 23 Tage zu verabreichenden Kombinationspräparates zur oralen Kontrazeption gegenüber den üblichen 21 Tage-Präparaten mit weniger als 30 µg Ethinylestradiol lassen sich wie folgt charakterisieren:
1. Eine signifikant geringere Häufigkeit von Follikelentwicklungen bei der Anwenderin (max. 13% bei Frauen, die das 23 Tage-Präparat gegenüber max. 40% bei denen, die das 21 Tage-Präparat erhielten). Dies bedeutet eine größere kontrazeptive Zuverlässigkeit des 23 Tage-Präparates, insbesondere bei vorhergegangenen Einnahmefehlern, Die Gefahr von "Durchbruchsovulationen" ist geringer.
2. Das Auftreten von großen Follikeln von mehr als 30 mm Durchmesser ist äußerst selten. Die Entwicklung von ovariellen Zysten ist unter dem 23 Tage-Präparat im Vergleich zum 21 Tage-Präparat unwahrscheinlich.
3. Die Rekrutierung von dominanten Follikeln wird in der verkürzten einnahmefreien Pause unterdrückt.
4. Die endogenen 1 7β-Estradiolspiegel werden beim überwiegenden Teil der Anwenderinnen des 23 Tage-Präparates gut kontrollierbar supprimiert. Klinische Symptome wie Brustspannungen, das prämenstruelle Syndrom und Blutungsstörungen, die auf erhöhte und stark fluktuierende Estrogenspiegel zurückzuführen sind, werden unter dem 23 Tage-Präparat mit deutlich geringerer Häufigkeit beobachtet.

Zusammengefaßt kann eine um zwei (oder drei) Tage verlängerte Einnahme von in jeder täglichen Dosierungseinheit 20 µg Ethinylestradiol enthaltenden Präparaten die genannten Vorteile erbringen, ohne daß die Tages-Dosis auf das bisher weitgehend verwendete Niveau von 30 *µ*g Ethinylestradiol angehoben werden muß.

Vorstehend genannte Vorteile, insbesondere eine bessere Unterdrückung der Follikelanreifung, lassen sich gemäß vorliegender Erfindung mit einem monophasischen Kombinationspräparat erreichen. Gegenüber einem mehrphasischen Präparat zeichnet sich ein monophasisches Präparat durch verschiedene Vorteile aus:
1. leichtere Herstellbarkeit
2. keine Pillenvertauschung aufgrund Nichtbeachtung der Einnahmereihenfolge
3. Menstruationsverschiebungen sind einfacher zu erreichen
4. die Einnahmehinweise sind für die Anwenderin leichter verständlich
5. die Packung bzw. der die Dosierungseinheiten enthaltende Blister braucht nicht mit einem Label zur Beachtung der Einnahmereihenfolge versehen zu sein.

Die Formulierung eines Estrogens und Gestagens für die erfindungsgemäße Verwendung oder für ein erfindungsgemäßes Kombinationspräparat erfolgt vollkommen analog wie es bereits für herkömmmliche orale Kontrazeptiva mit 21 tägiger Einnahmedauer der Wirkstoffe wie beispielsweise Femovan^{(R)} (Ethinylestradiol/Gestoden) oder Microgynon^{(R)} (Ethinylestradiol/Levonorgestrel) bekannt ist.

Eine ein erfindungsgemäßes Kombinationbspräparat enthaltende Packung ist ebenfalls analog wie Packungen für bereits bekannte, am Markt befindliche orale Kontrazeptiva aufgebaut mit der Abweichung, daß anstelle der üblichen 21, die aktiven Bestandteile enthaltenden. Dosierungseinheiten,nunmehr 23 oder 24 derartige Dosierungseinheiten und 5 oder 4 Blindpillen vorhanden sind oder aber andere geeignete Hinweise enthalten, daß 5 oder 4 Tage bis zur Fortsetzung der Einnahme wirkstoffholtiger Dosierungseinheiten zu überbrücken sind.

Im übrigen wird auf die in der EP-A 0 253 607 gemachten Angaben verwiesen, insbesondere auch auf die Angaben dort zur Bestimmung equivalenter Mengen von Ethinylestradiol und 17β-Estradiol einerseits und verschiedener Gestagene wie Levonorgestrel, Desogestrel, 3-Ketodesogestrel und Gestoden andererseits.

Für weitere Einzelheiten zur Bestimmung von Dosisequivalenten verschiedener gestagener Wirkstoffe wird verwiesen auf "Probleme der Dosisfindung: Sexualhormone"; F. Neumann et al. in "Arzneimittelforschung" (Drug Research) 27, 2a, 296 - 318 (1977) sowie auf "Aktuelle Entwicklungen in der hormonalen Kontrazeption"; H. Kuhl in "Gynäkologe" 25: 231 - 240 (1992).

## Patentansprüche

1. Verwendung einer oralen Dosierungsform umfassend ein Estrogen ausgewählt aus
2,0 bis 6,0 mg 17β-Estradiol, und
0,015 bis 0,020 mg Ethinylestradiol;
und ein Gestagen ausgewählt aus
0,05 bis 0,075 mg Gestoden,
0,075 bis 0,125 mg Levonorgestrel,
0,06 bis 0,15 mg Desogestrel,
0,06 bis 0,15 mg 3-Ketodesogestrel,
0,2 bis 0,3 mg Norgestimat,
>0,35 bis 0,75 mg Norethisteron,
0,1 mg Drospirenon bis zu einer zu 0,075 mg Gestoden equivalenten Dosis von Drospirenon, und
0,1 mg Cyproteronacetat bis zu einer zu 0,075 mg Gestoden equivalenten Dosis von Cyproteronacetat;
für die Empfängshisverhütung für eine Frau im fertilen Alter, die die Praemenopause noch nicht erreicht hat, durch Verabreichung der Dosierungsform während 23 oder 24 Tagen, beginnend am Tag eins des Menstruationszyklus, gefolgt von 5 oder 4 pillenfreien oder Blindpillentagen, während insgesamt 28 Tagen im Verabreichungszyklus.

2. Verwendung nach Anspruch 1, wobei das Estrogen Ethinylestradiol ist.

3. Verwendung nach Anspruch 1, wobei das Estrogen 17β-Estradiol ist.

4. Verwendung nach Anspruch 1, 2 oder 3, wobei das Gestagen Gestoden ist.

5. Verwendung nach Anspruch 1, 2 oder 3, wobei das Gestagen Levonorgestrel ist.

6. Verwendung nach Anspruch 1, 2 oder 3, wobei das Gestagen Cyproteronacetat oder Drospirenon ist.

7. Verwendung nach Anspruch 1, wobei die Dosierungsform ein Estrogen ausgewählt aus
>2,0 bis 6,0 mg 17β-Estradiol, und
0,020 mg Ethinylestradiol;
und ein Gestagen ausgewählt aus
>0,06 bis 0,075 Gestoden
>0,100 bis 0,125 mg Levonorgestrel
>0,10 bis 0,15 mg Desogestrel
> 0,10 bis 0,15 mg 3-Ketodesogestrel,
0,25 mg Drospirenone bis zu einer zu 0,075 mg Gestoden equivalenten Dosis von Drospirenon,
0,1 mg Cyproteronacetat bis zu einer zu 0,075 mg Gestoden equivalenten Dosis von Cyproteronacetat,
0,2 bis 0,3 mg Norgestimat und
0,50 bis 0,75 mg Norethisteron
umfaßt.

8. Verwendung nach Anspruch 1, wobei das Estrogen in einer Dosis von 20 µg Ethinylestradiol oder einer equivalenten Dosis von 17β-Estradiol und das Gestagen in einer Dosis von 75 µg Gestoden oder einer equivalenten Dosis von Levonorgestrel, Cyproteronacetat oder Drospirenon vorliegt.

9. Kombinationsprodukt für die orale Kontrazeption, weiches
(a) 23 oder 24 Dosierungseinheiten, jeweils enthaltend ein Estrogen ausgewählt aus
>2,0 bis 6,0 mg 17β-Estradiot, und
0,020 mg Ethinylestradial;
und ein Gestagen ausgewählt aus
0,25 mg Drospirenon bis zu einer zu 0,075 mg Gestoden equivalenten Dosis von Drospirenon, und
0,1 mg Cyproteronacetat bis zu einer zu 0,075 mg Gestoden equivalenten Dosis von Cyproteronacetat;
und
(b) 5 oder 4 Blindpillen oder andere Indikationen, um anzuzeigen, daß die tägliche Verabreichung der 23 oder 24 Dosierungseinheiten von 5 oder 4 pillenfreien oder Blindplllentagen gefolgt sein soil, umfaßt.

10. Kombinationspräparat nach Anspruch 9, wobei das Estrogen Ethinylestradiol ist.

11. Kombinationspräparat nach Anspruch 9, wobei das Estrogen 17β-Estradiol ist.

12. Kombinationspräparat nach einem der Ansprüche 9 bis 11, wobei das Gestagen Drospirenon ist.

13. Kombinationspräparat nach einem der Ansprüche 9 bis 11, wobei das Gestagen Cyproteronacetat ist.

14. Kombinationspräparat nach Anspruch 9, wobei das Estrogen Ethinylestradiol ist und wobei das Gestagen Drospirenon ist.

15. Kombinationspräparat nach Anspruch 9, wobei das Estrogen in einer Dosis von 20 µg Ethinylestradiol oder einer equivalenten Dosis von 17β-Estradiol und das Gestagen in einer Dosis von 75 µg Gestoden equivalenten Dosis von Cyproteronacetat oder Drospirenon vorliegt.

16. Monophasisches Kombinationspräparat nach einem der Ansprüche 9 bis 14, welches 23 Dosierungseinheiten und 5 Blindpillen oder andere indikationen, um anzuzeigen, daß keine Dosierungseinheit oder eine Blindpille während der letzten 5 Tage des Menstruationszyklus verabreicht wird, umfaßt.

17. Monophasisches Kombinationspräparat nach Anspruch 9, welches 23 Dosierungseinheiten, jeweils enthaltend 20 µg Ethinylestradiol und 75 µg Gestoden equivalenten Dosis von Drospirenon und 5 Blindpillen oder andere Indikationen, um anzuzeigen, daß keine Dosierungseinheit oder eine Blindpille während der letzten 5 Tage des Menstruationszyklus verabreicht wird, umfaßt.

18. Monophasisches Kombinationspräparat nach einem der Ansprüche 9 bis 14, welches 24 Dosierungseinheiten und 4 Blindpillen oder andere indikationen, um anzuzeigen, daß keine Dosierungseinheit oder eine Blindpille während der letzten 4 Tage des Menstruationszyklus verabreicht wird, umfaßt.

19. Monophasisches Kombinationspräparat nach Anspruch 9, welches 24 Dosierungseinheiten, jeweils enthaltend 20 µg Ethinylestradiol und 75 µg Gestoden equivalenten Dosis von Drospirenon und 4 Blindpillen oder andere Indikationen, um anzuzeigen, daß keine Dosierungseinheit oder eine Blindpille während der letzten 4 Tage des Menstruationszyklus verabreicht wird, umfaßt.

## Claims

1. Use of an oral dosage form comprising an oestrogen selected from
2.0 to 6.0 mg of 17β-estradiol, and
0.015 to 0.020 mg of ethinylestradiol;
and a progestogen selected from
0.05 to 0.075 mg of gestodene,
0.075 to 0.125 mg of levonorgestrel
0.06 to 0.15 mg of desogestrel
0.06 to 0.15 mg of 3-ketodesogestrel
0.2 to 0.3 mg of norgestimate,
> 0.35 to 0.75 mg of norethisterone,
0.1 mg of drospirenone to a drospirenone dose equivalent to 0.075 mg of gestodene, and
0.1 mg of cyproterone acetate to a cyproterone acetate dose equivalent to 0.075 mg of gestodene;
for contraception for a woman of fertile age who has not yet reached the premenopause, by administering the dosage form for 23 or 24 days, starting on day one of the menstrual cycle, followed by 5 or 4 pill-free or placebo pill days, for a total of 28 days in the administration cycle.

2. Use according to Claim 1, where the oestrogen is ethinylestradiol.

3. Use according to Claim 1, where the oestrogen is 17β-estradiol.

4. Use according to Claim 1, 2 or 3, where the progestogen is gestodene.

5. Use according to Claim 1, 2 or 3, where the progestogen is levonorgestrel.

6. Use according to Claim 1, 2 or 3, where the progestogen is cyproterone acetate or drospirenone.

7. Use according to Claim 1, where the dosage form comprises an oestrogen selected from
> 2.0 to 6.0 mg of 17β-estradiol, and
0.020 mg of ethinylestradiol;
and a progestogen selected from
> 0.06 to 0.075 of gestodene
> 0.100 to 0.125 mg of levonorgestrel
> 0.10 to 0.15 mg of desogestrel
> 0.10 to 0.15 mg of 3-ketodesogestrel
0.25 mg of drospirenone to a drospirerone dose equivalent to 0.075 mg of gestodene
0.1 mg of cyproterone acetate to a cyproterone acetate dose equivalent to 0.075 mg of gestodene,
0.2 to 0.3 mg of norgestimate and
0.50 to 0.75 mg of norethisterone.

8. Use according to Claim 1, where the oestrogen is present in a dose of 20 µg of ethinylestradiol or of an equivalent dose of 17β-estradiol and the progestogen is present in a dose of 75 µg of gestodene or of an equivalent dose of levonorgestrel, cyproterone acetate or drospirenone.

9. Combination product for oral contraception which comprises
(a) 23 or 24 dosage units, each comprising an oestrogen selected from
> 2.0 to 6.0 mg of 17β-estradiol, and
0.020 mg of ethinylestradiol;
and a progestogen selected from
0.25 mg of drospirenone to a drospirenone dose equivalent to 0.075 mg of gestodene, and
0.1 mg of cyproterone acetate to a cyproterone acetate dose equivalent to 0.075 mg of gestodene;
and
(b) 5 or 4 placebo pills or other indications in order to indicate that the daily administration of the 23 or 24 dosage units is to be followed by 5 or 4 pill-free or placebo pill days.

10. Combination products according to Claim 9, where the oestrogen is ethinylestradiol.

11. Combination product according to Claim 9, where the oestrogen is 17β-estradiol.

12. Combination product according to any of Claims 9 to 11, where the progestogen is drospirenone.

13. Combination product according to any of Claims 9 to 11, where the progestogen is cyproterone acetate.

14. Combination product according to Claim 9, where the oestrogen is ethinylestradiol and where the progestogen is drospirenone.

15. Combination product according to Claim 9, where the oestrogen is present in a dose of 20 µg of ethinylestradiol or of an equivalent dose of 17β-estradiol and the progestogen is present in a dose of 75 µg of gestodene or of an equivalent dose of cyproterone acetate or drospirenone.

16. Monophasic combination product according to any of Claims 9 to 14, which comprises 23 dosage units and 5 placebo pills or other indications in order to indicate that no dosage unit or a placebo pill is administered during the last 5 days of the menstrual cycle.

17. Monophasic combination product according to Claim 9, which comprises 23 dosage units, each comprising 20 µg of ethinylestradiol and 75 µg of gestodene equivalent dose of drospirenone and 5 placebo pills and other indications in order to indicate that no dosage unit or a placebo pill is administered during the last 5 days of the menstrual cycle.

18. Monophasic combination product according to any of Claims 9 to 14, which comprises 24 dosage units and 4 placebo pills or other indications in order to indicate that no dosage unit or a placebo pill is administered during the last 4 days of the menstrual cycle.

19. Monophasic combination product according to Claim 9, which comprises 24 dosage units, each comprising 20 µg of ethinylestradiol and 75 µg of gestodene equivalent dose of drospirenone and 4 placebo pills and other indications in order to indicate that no dosage unit or a placebo pill is administered during the last 4 days of the menstrual cycle.

## Revendications

1. Utilisation d'une forme posologique orale comprenant un estrogène choisi parmi :
2,0 à 6,0 mg de 17β-estradiol, et
0,015 à 0,020 mg d'éthinylestradiol ;
et un gestagène choisi parmi :
0,05 à 0,075 mg de gestodène,
0,075 à 0,125 mg de lévonorgestrel,
0,06 à 0,15 mg de désogestrel,
0,06 à 0,15 mg de 3-cétodésogestrel,
0,2 à 0,3 mg de norgestimate,
plus de 0,35 à 0,75 mg de noréthistérone,
0,1 mg de drospirénone à une dose de drospirénone équivalente à 0,075 mg de gestodène, et
0,1 mg d'acétate de cyprotérone à une dose d'acétate de cyprotérone équivalente à 0,075 mg de gestodène ;
pour la contraception d'une femme en âge de procréer qui n'est pas encore préménopausée, en prenant la forme posologique pendant 23 ou 24 jours, en commençant au premier jour du cycle menstruel avec ensuite 5 ou 4 jours sans comprimés ou avec des comprimés placebo sur un total de 28 jours pendant le cycle de prise.

2. Utilisation selon la revendication 1, dans laquelle l'estrogène est l'éthinylestradiol.

3. Utilisation selon la revendication 1, dans laquelle l'estrogène est le 17β-estradiol.

4. Utilisation selon la revendication 1, 2 ou 3, dans laquelle le gestagène est le gestodène.

5. Utilisation selon la revendication 1, 2 ou 3, dans laquelle le gestagène est le lévonorgestrel.

6. Utilisation selon la revendication 1, 2 ou 3, dans laquelle le gestagène est l'acétate de cyprotérone ou la drospirénone.

7. Procédé selon la revendication 1, dans lequel la forme posologique comprend un estrogène choisi parmi :
plus de 2,0 à 6,0 mg de 17β-estradiol, et
0,020 mg d'éthinylestradiol ;
et un gestagène choisi parmi :
plus de 0,06 à 0,075 mg de gestodène,
plus de 0,100 à 0,125 mg de lévonorgestrel,
plus de 0,10 à 0,15 mg de désogestrel,
plus de 0,10 à 0,15 mg de 3-cétodésogestrel,
0,25 mg de drospirénone à une dose de drospirénone équivalente à 0,075 mg de gestodène,
0,1 mg d'acétate de cyprotérone à une dose d'acétate de cyprotérone équivalente à 0,075 mg de gestodène,
0,2 à 0,3 mg de norgestimate, et
0,50 à 0,75 mg de noréthistérone.

8. Utilisation selon la revendication 1, dans laquelle l'estrogène est présent dans une dose de 20 µg d'éthinylestradiol ou une dose équivalente de 17β-estradiol et le gestagène est présent dans une dose de 75 µg de gestodène ou une dose équivalente de lévonorgestrel, d'acétate de cyprotérone ou de drospirénone.

9. Produit combiné pour contraception orale, lequel comprend :
(a) 23 ou 24 doses contenant chacune un estrogène choisi parmi :
plus de 2,0 à 6,0 mg de 17β-estradiol, et
0,020 mg d'éthinylestradiol ;
et un gestagène choisi parmi :
0,25 mg de drospirénone à une dose de drospirénone équivalente à 0,075 mg de gestodène, et
0,1 mg d'acétate de cyprotérone à une dose d'acétate de cyprotérone équivalente à 0,075 mg de gestodène ; et
(b) 5 ou 4 comprimés placebo ou d'autres indications pour indiquer la prise journalière des 23 ou 24 doses, les 5 ou 4 jours sans comprimés ou avec prise de comprimés placebo.

10. Préparation combinée selon la revendication 9, dans laquelle l'estrogène est l'éthinylestradiol.

11. Préparation combinée selon la revendication 9, dans laquelle l'estrogène est le 17β-estradiol.

12. Préparation combinée selon l'une des revendications 9 à 11, dans laquelle le gestagène est la drospirénone.

13. Préparation combinée selon l'une des revendications 9 à 11, dans laquelle le gestagène est l'acétate de cyprotérone.

14. Préparation combinée selon la revendication 9, dans laquelle l'estrogène est l'éthinylestradiol et dans laquelle le gestagène est la drospirénone.

15. Préparation combinée selon la revendication 9, dans laquelle l'estrogène est présent dans une dose de 20 µg d'éthinylestradiol ou une dose équivalente de 17β-estradiol et le gestagène est présent dans une dose de 75 µg de gestodène ou une dose équivalente d'acétate de cyprotérone ou de drospirénone.

16. Préparation combinée monophasique selon l'une des revendications 9 à 14, qui comprend la prise de 23 doses et de 5 comprimés placebo ou d'autres indications pour indiquer l'absence de prise de dose ou la prise d'un comprimé placebo pendant les 5 derniers jours du cycle menstruel.

17. Préparation combinée monophasique selon la revendication 9, qui comprend la prise de 23 doses contenant respectivement 20 µg d'éthinylestradiol et une dose de drospirénone équivalente à 75 µg de gestodène et la prise de 5 comprimés placebo ou d'autres indications pour indiquer l'absence de prise de dose ou la prise d'un comprimé placebo pendant les 5 derniers jours du cycle menstruel.

18. Préparation combinée monophasique selon l'une des revendications 9 à 14, qui comprend la prise de 24 doses et de 4 comprimés placebo ou d'autres indications pour indiquer l'absence de prise de dose ou la prise d'un comprimé placebo pendant les 4 derniers jours du cycle menstruel.

19. Préparation combinée monophasique selon la revendication 9, qui comprend la prise de 24 doses contenant respectivement 20 µg d'éthinylestradiol et une dose de drospirénone équivalente à 75 µg de gestodène et la prise de 4 comprimés placebo ou d'autres indications pour indiquer l'absence de prise de dose ou la prise d'un comprimé placebo pendant les 4 derniers jours du cycle menstruel.
